# EUROPÄISCHE PATENTSCHRIFT

(11) **EP 0 453 450 B1**
(45) Veröffentlichungstag und Bekanntmachung des Hinweises auf die Patenterteilung: **04.08.1993**
(21) Anmeldenummer: 90900180.2
(22) Anmeldetag: 08.01.1990
(51) Int. Cl.: H04R 25/00

(54) **FERNSTEUERBARES PROGRAMMIERBARES HÖRGERÄTESYSTEM**
REMOTE-CONTROLLED, PROGRAMMABLE HEARING AID SYSTEM
SYSTEME PROGRAMMABLE A TELECOMMANDE POUR APPAREILS DE CORRECTION AUDITIVE

(30) Priorität: 11.01.1989 DE 3900588
(43) Veröffentlichungstag der Anmeldung: 30.10.1991
(73) Patentinhaber: TOPHOLM & WESTERMANN APS, DK-3500 Vaerloese (DK)
(72) Erfinder: TOPHOLM, Jan, DK-2840 Holte (DK)
(74) Vertreter: Böhmer, Hans Erich, Dipl.-Ing.
(86) Internationale Anmeldenummer: EP9000031
(87) Internationale Veröffentlichungsnummer: WO9008448

(56) Entgegenhaltungen:
- EP-A- 0 064 042
- EP-A- 0 335 542
- DE-A- 3 642 828
- US-A- 4 791 672

## Beschreibung

Die Erfindung betrifft ein fernsteuerbares, programmierbares Hörgerätesystem gemäß dem Oberbegriff des Patentanspruchs 1.

Ein derartiges System ist beispielsweise aus der DE-A 36 42 828.0 bekanntgeworden.

Bei diesem System ist in dem eigentlichen Hörgerät in den einzelnen Stufen des Übertragungskanals vom Mikrofon zum Hörer eine Basis-Übertragungscharakteristik fest eingestellt. Andere Übertragungscharakteristika sind in einem externen Steuergerät abgespeichert und können wahlweise durch Betätigung eines Schalters oder einer Drucktaste ausgewählt und mit Hilfe des in dem externen Steuergerät eingebauten Senders an den in dem Hörgerät vorgesehenen Empfänger übertragen werden.

Diese von dem Hörgerät aufgenommenen Signale dienen nach Demodulation und entsprechender Verarbeitung der Einstellung einer anderen Übertragungscharakteristik des Hörgerätes zwischen Mikrofon und Hörer zur Anpassung an eine von mehreren im externen Steuergerät beispielsweise in Form von Steuerparametern digital abgespeicherten Umbebungssituationen.

Dieses an sich schon sehr gute System kann jedoch noch weiter verbessert werden, indem man ganz von vorgegebenen Übertragungsfunktionen sowohl im Hörgerät selbst als auch im Steuergerät abgeht und damit nicht nur die Vielseitigkeit des Gerätes erheblich verbessert sondern auch die Programmierung entscheidend vereinfacht.

Dies wird erfindungsgemäß durch die Merkmale des Patentanspruchs 1 erreicht.

Weitere Merkmale der Erfindung sind den weiteren Ansprüchen zu entnehmen.

Die Erfindung wird nunmehr anhand eines Ausführungsbeispieles in Verbindung mit den beigefügten Zeichnungen beschrieben.

Dabei zeigt:
- Fig. 1: schematisch ein vereinfachtes Blockschaltbild des erfindungsgemäßen Hörgerätes;
- Fig. 2: schematisch ein externes Steuergerät
und
- Fig. 3: eine weitere Ausführungsform der Erfindung.

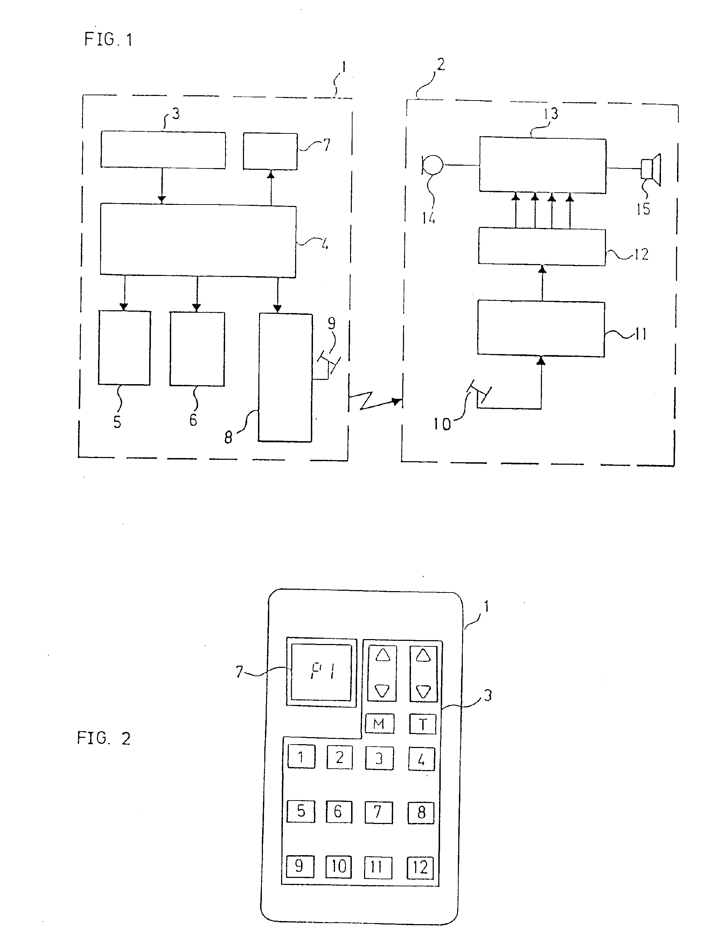

Figur 1 zeigt ein fernsteuerbares programmierbares Hörgerätesystem, das aus einem externen Steuergerät 1 und einem eigentlichen Hörgerät 2 besteht, das beispielsweise ein hinter dem Ohr zu tragendes Gerät oder ein Im-Ohr-Gerät sein kann.

Im-Ohr-Geräte können dabei auch unmittelbar an der Concha getragene Geräte sein.

Das Steuergerät kann dabei etwa die Form eines in der DE-A 36 42 828.0 gezeigten Gerätes aufweisen oder aber ein für den Hörgeräteakustiker bestimmtes Programmiergerät sein, mit dem sich dann das Steuergerät gemäß der oben genannten Patentanmeldung programmieren ließe.

Der Einfachheit halber sei zur Erläuterung das erste Beispiel gewählt.

Das Steuergerät 1 enthält eine Tastatur 3 mit einer Vielzahl von Tasten oder Drucktasten für die Eingabe von audiometrischen Daten/Informationen, Umgebungssituationen betreffende Daten/Informationen und zum Abruf der Daten/Informationen für eine Anzeige.

Die Tastatur 3 ist mit einem Datenprozessor 4 z.B. einem Microprozessoer verbunden, an dem ein erster Speicher 5 für Audiometriedaten und ein zweiter Speicher 6 für verschiedene Umgebungssituationen darstellende Daten angeschlossen sind.

Ferner ist an dem Datenprozessor 4 eine Anzeigevorrichtung 7, beispielsweise in Form einer Flüssigkristallanzeige angeschlossen. Schließlich ist mit dem Datenprozessor 4 noch ein Sender 8 verbunden, der für eine drahtlose Übertragung von Daten/Parametern an das eigentliche Hörgerät bestimmt ist. Zu diesem Zweck ist an dem Sender eine Antenne 9 vorgesehen.

Das Hörgerät weist eine Empfangsantenne 10 auf. Die dort aufgenommenen Signale werden in einem Empfänger 11 demoduliert und an ein Steuerparameter-Register 12 abgegeben. Dort werden die für eine bestimmte Übertragungscharakteristik bestimmten Parameter von der Seriendarstellung in eine Paralleldarstellung umgesetzt und den einzelnen Stufen eines Verstärkers und Signalprozessors 13 für die jeweilig vorzunehmende Einstellung der Übertragungscharakteristik zwischen Mikrofon 14 und Hörer 15 zugeführt.

Das Steuergerät 1 mit seiner Tastatur 3 dient, wie bereits erwähnt, einmal der Eingabe der audiometrischen Daten eines Patienten. Diese Daten betreffen jeweils den Hörverlust bei einer Anzahl diskreter Frequenzen, z.B. bei 125 Hz, 250 Hz, 500 Hz, 1000 Hz, 2000 Hz usw.

Jeder dieser Werte wird mittels der Tastatur 3 etwa in der Weise eingegeben, daß für jede Frequenz durch schrittweises Fortschalten, das an der durch den Mikroprozessor gesteuerten Anzeigevorrichtung 7 verfolgt werden kann, dieser Wert so lange automatisch verändert wird, bis der gewünschte Eingabewert auf der Anzeige erscheint. Dann wird dieser Wert als Steuerparameter festgehalten und in den Speicher 5 abgespeichert. Mit den übrigen Werten verfährt man genauso bis das gesamte Audiogramm im Speicher 5 liegt.

Mit diesen so ermittelten Parameterwerten läßt sich die Übertragungscharakteristik das Hörgerätes für den Normalfall einstellen.

Es ist bekannt, daß eine solche Grundeinstellung für die unterschiedlichsten Umgebungssituationen nicht geeignet ist. Daher kann man bei dem neuen Hörgerät in der gleichen Weise Parameter für verschiedene Umgebungssituationen mit Hilfe der Tastatur 3 unter Verwendung der Anzeigevorrichtung 7 und der datenverarbeitenden Vorrichtung 4 errechnen oder ableiten und im Speicher 6 abspeichern.

Wenn dann das Hörgerät mit diesem externen Steuergerät betrieben werden soll, dann müssen auch mehrere Programmtasten vorgesehen sein. Wenn dann eine dieser Programmtasten gedrückt wird, ruft der Mikroprozessor die entsprechenden Daten aus dem Speicher 6 ab und modifiziert die im Speicher 5 abgespeicherten Daten zur Ermittlung neuer für diese spezielle Umgebungssituation bestimmte Parameter. Diese werden dann über den Sender 8 nach dem Empfänger 11 des Hörgerätes übertragen und in Steuerparameter für den Verstärker und Signalprozessor umgesetzt. Das Hörgerät ist jetzt für diesen Patienten und für eine bestimmte Umgebungssituation eingestellt.

Durch Betätigen einer anderen Programmtaste kann dann das Hörgerät auf eine andere Umgebungssituation umgstellt werden. Ebenso ist es möglich, das Hörgerät wieder auf die durch die Audiometrie bestimmte Grundeinstellung einzustellen.

Die Programmiervorrichtung kann auch in einem für den Hörgeräteakustiker bestimmten Programmiergerät untergebracht sein, das dann für die Programmierung eines Hörgerätes eingesetzt werden kann, wie es in der oben genannten Patentanmeldung DE-A 36 42 828.0 beschrieben ist.

Auf diese Weise läßt sich das neue Hörgerät jederzeit auch auf geänderte Audiometriedaten des Patienten neu programmieren. Außerdem können auch an Stelle der bereits abgespeicherten Daten für Umgebungssituationen andere Daten dieser Art eingegeben und abgespeichert werden. Dadurch wird dieses Gerät in vielfacher Weise verwendbar.

Die Fig. 2 zeigt nun rein schematisch ein solches externes Steuergerät, das ähnlich aufgebaut sein kann wie das Steuergerät gemäß der oben genannten Patentanmeldung.

Dieses Steuergerät 1 weist auf seiner Oberfläche eine Anzeigevorrichtung 7, z.B. in Form einer Flüssigkeitskristallanzeige sowie eine Tastatur 3 auf.

Die mit Pfeilen gekennzeichneten Tasten der Tastatur 3 dienen der erwähnten Fortschaltung bei der Eingabe von Audiometriedaten. In dieser Ausführungsform dient der linke Schalter für ein für das linke Ohr bestimmtes Hörgerät und der rechte Schalter für ein für das rechte Ohr bestimmtes Hörgerät. Die Taste M dient zum Anschalten des Mikrofons im Hörgerät und die Taste T für das Anschalten einer Telefonspule. Die Taste 1 bis 4 können als Programmtasten, beispielsweise für den Abruf von vier verschiedenen Sätzen aus den Parameterspeichern 5 und/oder 6 bestimmt sein und dienen der Einleitung der Übertragung entsprechender Steuerparameter an das Hörgerät.

Die Anordnung kann z.B. so getroffen sein, daß mit den Programmtasten eine im Speicher 6 gespeicherte, eine bestimmte Umgebungssituation berücksichtigende Gruppe von Steuerparametern zur Modifizierung der im Parameterspeicher 5 gespeicherten Normalparameter abgerufen werden kann. Damit lassen sich einige Umgebungssituationen berücksichtigen.

Dabei werden z.B. mit den Parametern aus dem Parameterspeicher 6 durch den Mikroprozessor die Parameter der Normaleinstellung aus dem Speicher 5 modifiziert und die so modifizierten Parameter zur Einstellung der neuen Übertragungscharakteristik des Hörgerätes an dieses übertragen. D.h. auf einer der Tasten 1 bis 4 liegt die Normaleinstellung, denn das Hörgerät selbst hat keine permanente Einstellung einer Übertragungscharakteristik, sondern immer nur die Einstellung, die einer der Programmtasten entspricht.

Da die in den Speichern 5 und 6 gespeicherten Parameter jederzeit bei Bedarf durch den Hörgeräteakustiker umprogrammiert werden können, ist dieses Gerät praktisch an jede neue Situation anpaßbar, was sowohl die Grundeinstellung als auch die Parameter für verschiedene Umgebungssituationen betrifft.

Die in Fig. 2 gezeigten weiteren Tastaturreihen mit jeweils weiteren vier Tasten sollen nur symbolisch die Eingabemittel für die Audiometriedaten und für die Daten für spezifische Umgebungssituationen darstellen. Die Belegung der einzelnen Tasten und ihre Anzahl und Anordnung wird man selbstverständlich den jeweiligen Bedürfnissen anpassen und sie sind nicht Gegenstand der Erfindung.

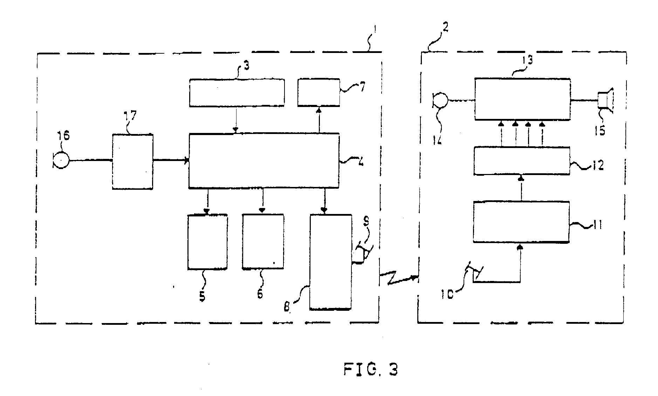

Die wohl eleganteste Variante des neuen Hörgerätes läßt sich mit Hilfe eines in dem externen Steuergerät gemäß Fig. 3 eingebauten zusätzlichen Mikrofons 16 erzielen. Dieses Mikrofon kann beispielsweise durch eine der vier Programmtasten, z.B. Programmtaste 4, anschaltbar sein.

Über dieses Mikrofon wird die augenblickliche Umgebungssituation, also z.B. die Geräusche eines Wohnzimmers, eines Abteils in einem Zug der Eisenbahn oder Straßenbahn oder bei einem Empfang mit vielen Teilnehmern als Audiosignal aufgenommen und einer Analysier- und Auswerteschaltung 17 zugeführt. Diese Schaltung analysiert dieses Signal nach Energiegehalt über den ganzen hier in Frage kommenden Frequenzbereich und ermittelt mittels eines oder mehrerer gespeicherter Algorithmen mit Hilfe des Mikroprozessors der datenverarbeitenden Vorrichtung 4 in Verbindung mit den aus den audiometrischen Daten abgeleiteten Steuerparametern einen dem aufgenommenen Audiosignal entsprechenden Satz von Steuerparametern zur Übertragung an das Hörgerät.

Schließlich kann man noch einen Schritt weiter gehen und die Programmtasten sowie die Eingabetasten für Umgebungssituationen und den Speicher für Umgebungsparameter wegfallen lassen.

In diesem Fall enthält das externe Steuergerät neben der Eingabetastatur für die Audiometriedaten einen Speicher für die daraus abgeleiteten Steuerparameter für die Grundeinstellung. Alle anderen Einstellungen werden dann automatisch über das externe Steuergerät und das darin eingebaute Mikrofon 16 durch Analyse des Klangbildes oder Geräuschbildes durch den Mikroprozessor mit Hilfe von gespeicherten Algorithmen ermittelt, berechnet oder abgeleitet und unmittelbar zur Steuerung der Übertragungscharakteristik des Hörgerätes verwendet.

Diese Algorithmen können entweder in einem Festwertspeicher außerhalb oder innerhalb der datenverarbeitenden Vorrichtung gespeichert sein oder sie können auch in einem dynamischen flüchtigen Speicher liegen, der entweder an die datenverarbeitende Vorrichtung angeschlossen ist oder in dieser selbst enthalten sein kann. In diesem Fall können die Algorithmen bei Bedarf verändert werden.

Damit ist sichergestellt, daß das Hörgerät für jede beliebige Umgebungssituation unmittelbar einsetzbar ist und sich beim Übergang in eine andere Umgebungssituation automatisch auf diese neue Situation einstellt.

Bei einer derartigen Anordnung kann man schließlich cie Bedienung des neuen Hörgerätes vollständig über die Anzeigevorrichtung steuern, indem man alle zur Bedienerführung vorgesehenen Informationen auf der Anzeigevorrichtung darstellen kann.

Damit ist gezeigt, daß durch die Erfindung das bekannte Hörgerät ganz entscheidend verbessert worden ist.

## Patentansprüche

1. Fernsteuerbares, programmierbares Hörgerätesystem, bestehend aus einem externen Steuergerät (1) mit einer Eingabevorrichtung (3) und einer Anzeigevorrichtung (7) zur Bedienerführung sowie mit einem Sender für die wahlweise Übertragung unterschiedlicher Gruppen von Steuerparametern aus einem Speicher an ein Hörgerät mit einer Empfangsschaltung, mit einem Verstärker und mit einer Signalverarbeitungsschaltung, deren Übertragungscharakteristik zu jedem beliebigen Zeitpunkt wahlweise durch einen vom externen Steuergerät übertragenen Satz von Steuerparametern einstellbar ist, dadurch gekennzeichnet, daß das Fernsteuergerät (1) einen ersten Speicher (5) für die Aufnahme und Speicherung von audiometrischen Daten, einen zweiten Speicher (6) für die Aufnahme und Speicherung von unterschiedliche Umgebungssituationen kennzeichnenden Daten, sowie eine datenverarbeitende Varrichtung (4) für die Ermittlung der Gruppen von Steuerparametern aus den audiometrischen Daten und den die Umgebungssituationen kennzeichnenden Daten gemäß einem oder mehrerer Algorithmen enthält, die in einem dritten Speicher in Verbindung mit der datenverarbeitenden Vorrichtung gespeichert sind.

2. Hörgerätesystem nach Anspruch 1, dadurch gekennzeichnet, daß in dem externen Steuergerät (1) als datenverarbeitende Vorrichtung (4) ein Mikroprozessor vorgesehen ist, der einerseits mit der Eingabevorrichtung (3) und der Anzeigevorrichtung (7) sowie mit dem Speicher (5) für die audiometrischen Daten und dem Speicher (6) für unterschiedliche Umgebungssituationen kennzeichnende Daten verbunden ist, und daß der Sender (8) an dem Mikroprozessar angeschlossen ist.

3. Hörgerätesystem nach Anspruch 1 oder 2, dadurch gekennzeichnet, daß wenigstens einer der Datenspeicher (5, 6) und der die Algorithmen enthaltenden Speicher in den Mikroprozessor integriert sind.

4. Hörgerätesystem nach einem der Ansprüche 1 bis 3, wobei das externe Steuergerät (1) mit einem zusätzlichen Mikrofon (16) und mit einer Analysier- und Auswerteschaltung (17) versehen ist, dadurch gekennzeichnet, daß die datenverarbeitende Varrichtung (4) mit Hilfe eines oder mehrerer Algorithmen aus dem Ausgangssignal der Analysierund Auswerteschaltung (17) der augenblicklichen Umgebungssituation entsprechende Steuerparameter zur Übertragung an das Hörgerät ableitet.

5. Hörgerätesystem nach Anspruch 4, dadurch gekennzeichnet, daß die Analysier- und Auswerteschaltung (17) ein Teil der datenverarbeitenden Vorrichtung ist.

6. Hörgerätesystem nach einem der Ansprüche 1 bis 5, dadurch gekennzeichnet, daß eine Anzeigevorrichtung (7) zur Anzeige von Daten/ Informationen, die in den Speichermitteln des Hörgerätesystems gespeichert sind oder zur Anzeige von Daten0Informationen, die durch die datenverarbeitende Vorrichtung (4) bearbeitet, verarbeitet oder abgeleitet worden sind, sowie zur Anzeige von zur Bedienerführung vorgesehenen Informationen vorgesehen ist.

## Claims

1. Remote-controllable, programmable hearing aid system, consisting of an external control unit (1) with an input device (3) and display (7) for operator prompting and with a transmitter for optional transmission of different groups of control parameters from a memory to a hearing aid with a receiving circuit, amplifier and a signal processing circuit whose transmission characteristic can be optionally adjusted at any time by a set of control parameters transmitted by an external control unit, characterized in that the remote control unit (1) contains a first memory (5) for recording and storing audiometric data, a second memory (6) recording and storing data characterizing different environmental situations, as well as a data processing device (4) for determination of the groups of control parameters from audiometric data and data characterizing the environmental situations in accordance with one or more algorithms, the latter being stored in a third memory in conjunction with the data processing device.

2. Hearing aid system in accordance with Claim 1, characterized in that a microprocessor is provided as a data processing device (4) in the external control unit (1), whereby this microprocessor is connected with the input device (3) and display (7) as well as with the memory (5) for audiometric data and the memory (6) for data characterizing different environmental situations, and in that the transmitter (8) is connected to the microprocessor.

3. Hearing aid system in accordance with Claim 1 or 2, characterized in that at least one of the data memories (5, 6) and the memory containing the algorithms are integrated in the microprocessor.

4. Hearing aid system in accordance with one of Claims 1 to 3, whereby the external control unit (1) is equipped with an additional microphone (16) and with an analysis and evaluation circuit (17), characterized in that the data processing device (4) derives control parameters corresponding to the current environmental situation for transmission to the hearing aid from the output signal of the analysis and evaluation circuit (17) with the assistance of one or more algorithms.

5. Hearing aid system in accordance with Claim 4, characterized in that the analysis and evaluation circuit (17) is part of the data processing device.

6. Hearing aid system in accordance with one of Claims 1 to 5, characterized in that a display (7) is provided for display of data/information stored in the storage media of the hearing aid system or for display of data/information which has been processed or derived by the data processing device (4) as well as for display of the information intended for operator prompting.

## Revendications

1. Système programmable à télécommande pour appareils de correction auditive comprenant un appareil externe de commande (1) avec un dispositif d'entrée (3) et un dispositif d'affichage (7) pour le guidage de l'utilisateur ainsi qu'avec un émetteur pour la transmission au choix de différents groupes de paramètres de commande d'une mémoire vers un appareil de correction auditive équipé d'un circuit récepteur, d'un amplificateur et d'un circuit de traitement de signaux dont les caractéristiques de transmission peuvent être déterminées à tout moment à l'aide d'un ensemble de paramètres de commande transmis par l'appareil externe de commande, caractérisé en ce que l'appareil de commande (1) contient une première mémoire (5) pour l'enregistrement et la mise en mémoire de données audiométriques, une seconde mémoire (6) pour l'enregistrement et la mise en mémoire de données caractéristiques de différentes situations environnantes ainsi qu'un dispositif (4) de traitement de données qui produit les groupes de paramètres de commande à partir des données audiométriques et des données caractéristiques des situations environnantes selon un ou plusieurs algorithmes mémorisés dans une troisième mémoire en liaison avec le dispositif de traitement de données.

2. Système pour appareils de correction auditive selon la revendication 1, caractérisé en ce qu'est prévu dans l'appareil externe de commande (1), en tant que dispositif de traitement de données (4), un microprocesseur relié, d'une part, au dispositif d'entrée (3) et au dispositif d'affichage (7) et, d'autre part, à la mémoire (5) pour les données audiométriques et à la mémoire (6) pour les données caractéristiques de différentes situations environnantes et en ce que l'émetteur (8) est relié au microprocesseur.

3. Système pour appareils de correction auditive selon l'une des revendications 1 ou 2, caractérisé en ce que l'une au moins des mémoires de données (5, 6) et la mémoire contenant les algorithmes sont intégrées au microprocesseur.

4. Système pour appareils de correction auditive selon l'une quelconque des revendications 1 à 3, l'appareil externe de commande (1) étant muni d'un microphone (16) supplémentaire et d'un circuit d'analyse et d'évaluation (17), caractérisé en ce que le dispositif (4) de traitement de données détermine à partir du signal de sortie du circuit d'analyse et d'évaluation (17), à l'aide d'un ou de plusieurs algorithmes, les paramètres de commande correspondants à la situation environnante momentanée pour la transmission à l'appareil de correction auditive.

5. Système pour appareils de correction auditive selon la revendication 4, caractérisé en ce que le circuit d'analyse et d'évaluation (17) fait partie du dispositif de traitement de données.

6. Système pour appareils de correction auditive selon l'une quelconque des revendications 1 à 5, caractérisé en ce qu'est prévu un dispositif d'affichage (7) pour l'affichage des données/informations mémorisées dans les supports de mémoire du système pour appareils de correction auditive ou pour l'affichage de données/informations ayant été mises en forme, traitées ou déterminées par le dispositif (4) de traitement de données ainsi que pour l'affichage d'informations destinées à guider l'utilisateur.
